# FASCICULE DE BREVET EUROPEEN

(11) **EP 1 443 879 B1**
(45) Date de publication et mention de la délivrance du brevet: **17.03.2010**
(21) Numéro de dépôt: 02788060.8
(22) Date de dépôt: 15.11.2002
(51) Int. Cl.: A61F 2/44

(54) **PROTHESE D'ARTICULATION VERTEBRALE POSTERIEURE**
GELENKSPROTHESE FÜR DEN HINTENLIEGENDEN WIRBELSÄULENBEREICH
POSTERIOR VERTEBRAL JOINT PROSTHESIS

(30) Priorité: 15.11.2001 FR 0114766
(43) Date de publication de la demande: 11.08.2004
(73) Titulaire: Louis, René, 13008 Marseille (FR); Tropiano, Patrick, 13008 Marseille (FR); Bronsard, Jean-Jacques, 13007 Marseille (FR); Louis, Christian, 13400 Aubagne (FR)
(72) Inventeur: Louis, René, 13008 Marseille (FR); Tropiano, Patrick, 13008 Marseille (FR); Bronsard, Jean-Jacques, 13007 Marseille (FR); Louis, Christian, 13400 Aubagne (FR)
(74) Mandataire: Domange, Maxime
(86) Numéro de dépôt international: PCT/FR2002/003911
(87) Numéro de publication internationale: WO 2003/041618

(56) Documents cités:
- WO-A-01/30248
- US-A- 5 571 191
- US-A1- 2002 065 557

## Description

La présente invention est relative à une prothèse d'articulation vertébrale postérieure.

Les douleurs vertébrales cervicales, dorsales et lombaires ont souvent pour origine les articulations vertébrales postérieures ou articulations zygapophysaires ou « facet joint ». Quand les thérapeutiques médicales ne suffisent plus pour les soulager, en particulier lorsque ces articulations sont très déformées par l'arthrose ou un traumatisme ancien, il devient souhaitable de remplacer leurs surfaces articulaires par des prothèses ; l'intérêt de ces prothèses est de conserver les fonctions et la mobilité de la colonne vertébrale, et d'éviter le blocage (arthrodèse) de ces articulations.

Il a été décrit dans les documents FR 2 721 501 et US 6 132 464 divers dispositifs de remplacement des surfaces articulaires des facettes vertébrales postérieures ; chacun de ces dispositifs comporte un support comportant une face convexe épousant et venant en appui au contact d'une portion de la surface concave de l'arc postérieur d'une vertèbre, d'un côté au moins de l'apophyse épineuse ; ce support est fixé à la vertèbre par une vis pédiculaire, et/ou aux apophyses épineuse et transverse par des moyens de cerclage tels qu'une bride, un collier, un crochet ; ce dispositif comporte en outre une lame formant une demi prothèse articulaire qui comporte deux faces opposées : une première face, dite articulaire ou de glissement, est en contact avec une face similaire d'une vertèbre adjacente avec un minimum de frottement ; cette première face est recouverte d'un matériau biocompatible à fort taux de glissement, tel que de l'acier inox ou du titane notamment ; une deuxième face opposée à la première face de la lame, dite face osseuse, est poreuse, parsemée de picots, recouverte ou non d'hydroxyapatite, et est destinée à venir en appui sur l'os restant de l'apophyse articulaire ; la lame est reliée au support par une base sessile, dont la face postérieure convexe et poreuse s'appuie sur l'os de l'arc vertébral ; les moyens de fixation du support sur les apophyses épineuse et transverse et sur le pédicule sont associés, en fonction des conditions anatomiques rencontrées, afin d'assurer la stabilité du montage.

Ces dispositifs présentent certains inconvénients ; du fait de la présence de nombreuses faces d'appui respectif des différentes parties (support, lame, base) du dispositif sur différentes régions d'une vertèbre, il est difficile d'assurer des appuis de qualité uniforme et satisfaisante en raison des variations de forme d'une vertèbre à une autre et/ou d'un patient à un autre ; en outre, la présence de la base, du support d'ancrage, et des divers moyens de fixation, contribue à augmenter le volume de la prothèse et par conséquent à augmenter la gêne pour le patient ainsi que les traumatismes consécutifs à l'implantation d'un tel dispositif.

Un objectif de l'invention est de proposer des prothèses d'articulation vertébrales postérieures qui soient améliorées et qui remédient, en partie au moins, aux inconvénients des prothèses de ce type.

Conformément à un premier aspect, l'invention est definie par la revendication 1 et consiste à proposer un dispositif comportant, pour l'articulation vertébrale postérieure gauche ou pour l'articulation vertébrale postérieure droite, une première prothèse de remplacement partiel de l'apophyse articulaire antérieure qui comporte une première surface d'appui glissant, ainsi qu'une deuxième prothèse de remplacement total de l'apophyse articulaire postérieure, qui comporte une deuxième surface d'appui glissant présentant au moins une portion de forme similaire ou identique à la forme d'au moins une portion de ladite première surface d'appui glissant de ladite première prothèse.

L'implantation de la première prothèse de remplacement partiel de l'apophyse articulaire antérieure nécessite une simple abrasion préalable de la surface (ou facette) articulaire de l'apophyse antérieure, tandis que l'implantation de la deuxième prothèse de remplacement total de l'apophyse articulaire postérieure est effectuée après une ablation sensiblement totale de l'apophyse postérieure.

Selon un autre aspect, l'invention consiste à proposer un dispositif de remplacement des surfaces articulaires vertébrales postérieures qui comporte, pour l'articulation gauche et pour l'articulation droite, une première prothèse d'apophyse articulaire antérieure comportant une première surface d'appui lisse, ainsi qu'une deuxième prothèse d'apophyse articulaire postérieure comportant une deuxième surface d'appui lisse dont la forme est complémentaire de celle de ladite première surface de la première prothèse, de façon à faciliter son glissement sur la première surface et à faciliter un mouvement relatif de rotation antéro-postérieure (ou sagittal) de deux vertèbres adjacentes munies de ces prothèses ; ceci permet aux vertèbres équipées des prothèses selon l'invention de conserver une mobilité importante.

Selon un autre aspect, l'invention consiste à proposer des prothèses d'articulation vertébrales postérieures présentant des surfaces lisses d'appui glissant qui épousent sensiblement la forme d'un dièdre : la première prothèse d'apophyse articulaire antérieure comporte une surface d'appui glissant en forme de dièdre concave dont les faces forment de préférence un premier angle situé dans une plage allant de 90° à 160°, tandis que la deuxième prothèse d'apophyse articulaire postérieure comporte une surface d'appui glissant en forme de dièdre convexe dont les faces forment un deuxième angle dont la valeur est égale ou voisine au complément à 360° de la valeur dudit premier angle.

La mise en contact mutuel desdites première et deuxième prothèse par leur surface lisse d'appui diédrique respective favorise le guidage mutuel des deux prothèses lors de leur déplacement mutuel de glissement, ce qui augmente la stabilité dynamique d'un rachis dont deux vertèbres adjacentes sont équipées de ces prothèses.

La valeur de l'angle d'ouverture des surfaces d'appui glissant diédriques est de préférence adaptée à la position de la vertèbre le long du rachis : pour l'articulation L5-S1 (lombo-sacrée), la valeur de l'angle de la prothèse articulaire antérieure est de préférence voisine de 90° à 160° et la valeur de l'angle de la prothèse articulaire postérieure est respectivement voisine de 270° à 200°, tandis que pour l'articulation L4-L5, l'angle de la prothèse antérieure est de préférence voisin de 90° à 150° et l'angle de la prothèse postérieure est respectivement voisin de 270° à 210°.

Selon un autre aspect, l'invention consiste à proposer des prothèses d'articulation vertébrales postérieures présentant des surfaces lisses d'appui glissant présentant une courbure antéro-postérieure : ladite première prothèse antérieure présente, (en vue médiale), une surface de glissement courbée ouverte vers l'arrière et ladite deuxième prothèse postérieure présente (en vue médiale) une surface de glissement courbée fermée vers l'avant, dont la courbure est égale à (ou voisine de) la courbure de la surface ouverte de la première prothèse ; ceci facilite la libre rotation relative antéro-postérieure de deux vertèbres adjacentes équipées de ces prothèses ; le rayon de courbure antéro-postérieure de ces surfaces de glissement peut être adapté à la position de la vertèbre à laquelle la prothèse est destinée ; la valeur de ce rayon est de préférence choisi dans une plage allant de 18 à 28 mm, de préférence encore de 20 à 26 mm, en particulier voisin de 23 mm.

Grâce à cette courbure, les mouvements de flexion-extension de la colonne vertébrale (rotation antéro-postérieure) peuvent être effectués selon un axe transversal commun pour le disque intervertébral et pour ces articulations.

Selon un mode préféré de réalisation, les surfaces de glissement présentent à la fois une forme diédrique et une courbure antéro-postérieure (sagittale) : la prothèse antérieure comporte une surface de glissement en forme de dièdre concave comportant une face médiale convexe vers l'arrière selon un rayon de courbure voisin de 20 à 26 mm, et une face latérale sensiblement plane ; la prothèse postérieure comporte une surface de glissement en forme de dièdre convexe comportant une face médiale concave vers l'avant selon un rayon de courbure voisin de 20 à 26 mm, et une face latérale sensiblement plane ; en d'autres termes, dans ce cas, lesdites surfaces de glissement s'étendent sensiblement selon une surface (et/ou le long d'une surface) de révolution engendrée par un segment incurvé tournant autour d'une droite (transverse) située dans un plan contenant le segment incurvé, à une distance de celui-ci qui est égale audit rayon de courbure ; ainsi les surfaces de glissement des prothèses gauche et droite épousent sensiblement des portions d'une forme de gorge torique (de révolution).

Selon un autre aspect, l'invention consiste à proposer une prothèse antérieure d'articulation vertébrale postérieure qui comporte une surface lisse d'appui glissant ainsi qu'une surface d'appui osseux, et une structure d'ancrage de la prothèse dans l'apophyse articulaire qui fait saillie sur ladite surface d'appui osseux ; l'invention propose également une prothèse postérieure d'articulation vertébrale postérieure comportant une surface d'appui glissant et une surface d'appui osseux, ainsi qu'une structure d'ancrage de la prothèse dans l'isthme vertébral et/ou dans la lame vertébrale, qui est saillante sur ladite face d'appui osseux.

L'invention permet d'obtenir des prothèses bien adaptées à l'anatomie humaine, compactes, stables et peu traumatisantes.

De préférence, chacune de ces structures d'ancrage comporte un organe d'ancrage allongé - tel qu'un ergot ou une tige - selon un axe qui est généralement incliné par rapport à la normale à la surface d'appui osseux ; de préférence ledit organe d'ancrage est de forme sensiblement cylindrique selon cet axe, et est fileté ou muni de rainures ou protubérances sur sa face externe, afin d'augmenter la surface de contact osseux.

Dans le cas notamment de la prothèse antérieure, l'ancrage résultant de cet organe peut être amélioré par la présence d'une nervure saillante le long d'une partie de la périphérie de la surface d'appui osseux, en augmentant la surface en contact avec l'os.

Dans le cas notamment de la prothèse postérieure, l'ancrage peut être amélioré par la présence de deux organes d'ancrage allongés - tels que des tiges ou ergots - selon deux axes sensiblement orthogonaux.

Lesdits organes d'ancrage allongés peuvent former une seule pièce avec le corps de prothèse comportant la face d'appui osseux et la face d'appui glissant ; alternativement ou en complément, un organe d'ancrage distinct peut être utilisé, en particulier sous forme d'une vis s'étendant dans un orifice prévu dans le corps de prothèse.

D'autres avantages et caractéristiques de l'invention seront compris au travers de la description suivante qui se réfère aux dessins annexés, qui illustrent sans aucun caractère limitatif des modes préférentiels de réalisation de l'invention.

La figure 1 est une vue dorsale schématique de la partie supérieure du sacrum et des vertèbres lombaires L4 et L5, sur laquelle sont représentées quatre prothèses articulaires postérieures gauche remplaçant deux articulations postérieures gauches superposées.

La figure 2 illustre schématiquement en vue craniale une prothèse articulaire antérieure gauche et une prothèse articulaire postérieure gauche remplaçant une articulation postérieure entre deux vertèbres successives du rachis.

La figure 3 illustre schématiquement en perspective la forme de la surface d'appui glissant de prothèses selon l'invention.

La figure 4 illustre schématiquement en vue latérale des vertèbres munies de prothèses articulaires et leur mode de déplacement mutuel en rotation selon un axe transverse.

Les figures 5 à 8 illustrent un mode de réalisation d'une prothèse articulaire antérieure adaptée à l'articulation lombo-sacrée L5-S1 et les figures 9 à 11 illustrent une prothèse articulaire postérieure complémentaire de celle des figures 5 à 8.

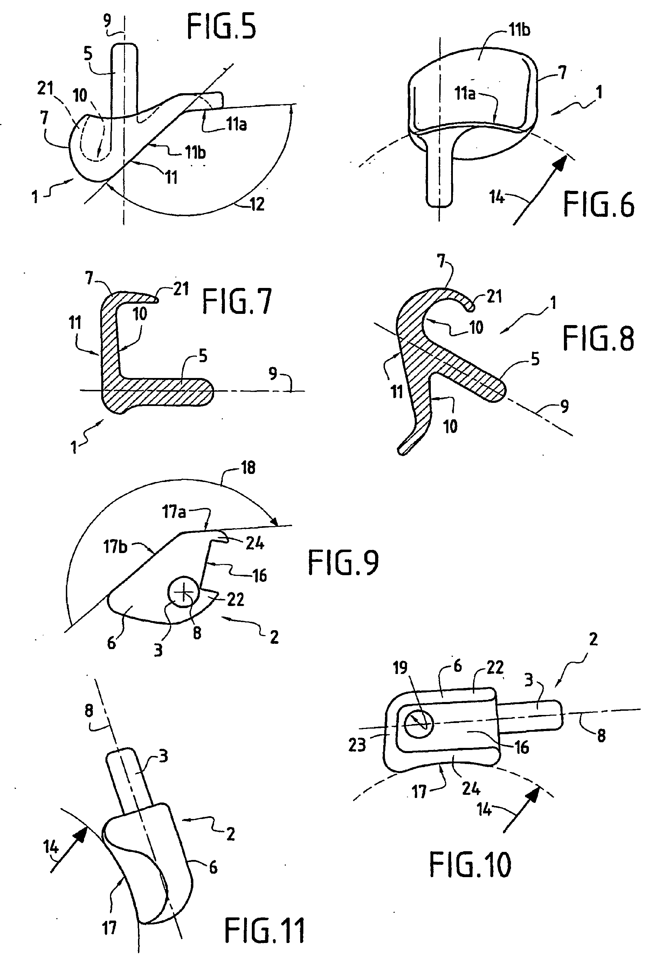

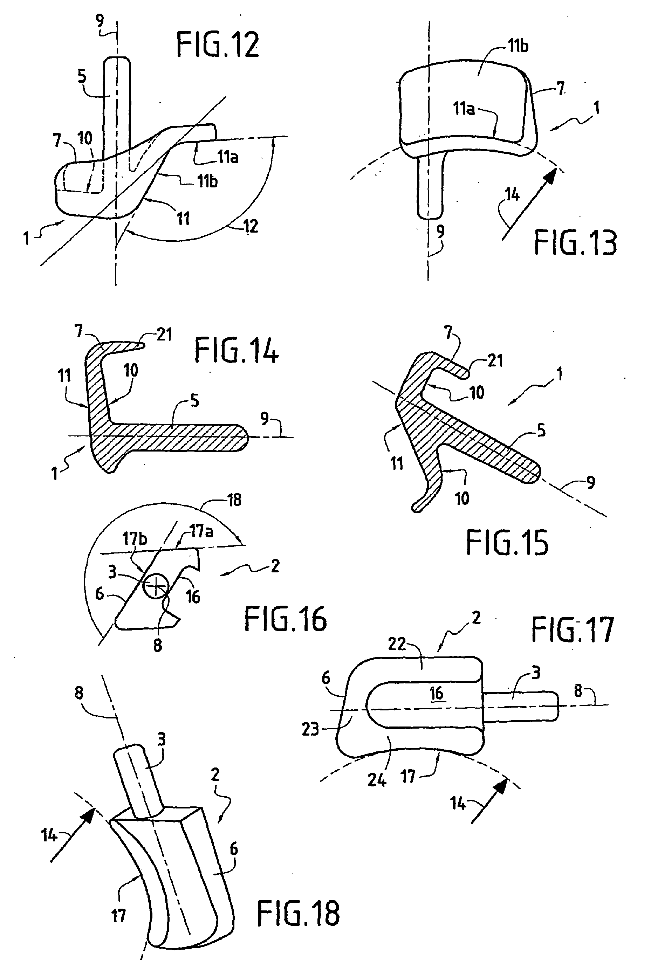

De la même façon que les figures 5 à 8, les figures 12 à 15 illustrent un mode de réalisation d'une prothèse articulaire antérieure adaptée pour l'articulation postérieure entre les lombaires L4 et L5, et les figures 16 à 18 illustrent une prothèse articulaire postérieure complémentaire de la prothèse des figures 12 à 15.

Les figures 5, 9, 12, 16 sont des vues craniales des prothèses ; les figures 6, 10, 13, 17 sont des vues médiales des prothèses ; les figures 7 et 14 sont des vues sagittales ; les figures 8 et 15 sont des vues horizontales ; les figures 11 et 18 sont des vues latérales.

Les figures 7, 8, 14, 15 sont des vues en coupe.

Bien que l'invention soit décrite ci-après pour la partie lombaire du rachis, l'invention est applicable aux articulations postérieures des vertèbres dorsales et cervicales.

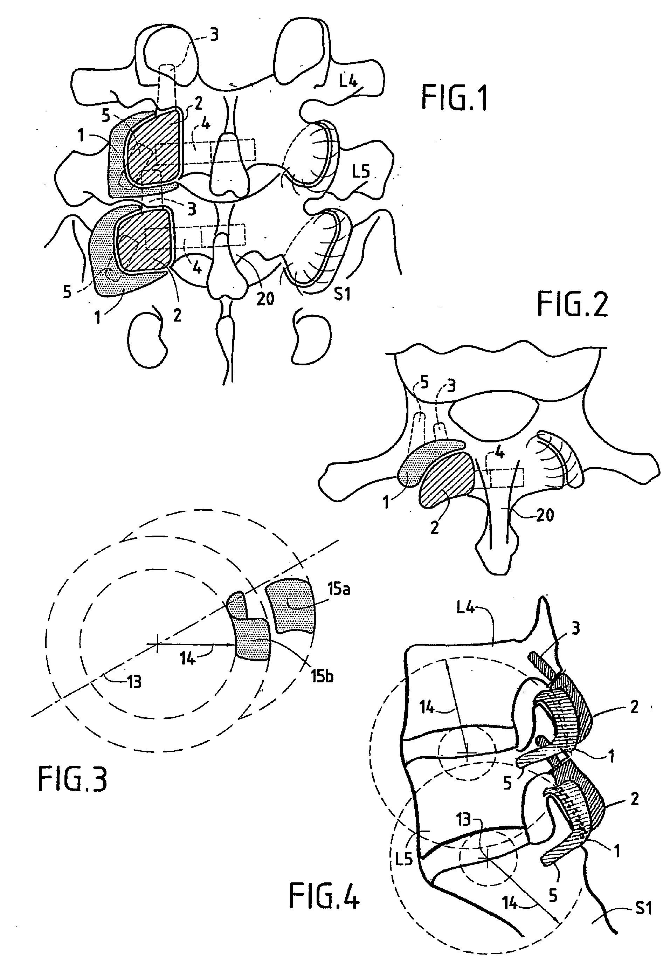

Par référence aux figures 1 et 2 en particulier, le remplacement d'une articulation vertébrale postérieure, gauche ou droite, est effectué un utilisant une prothèse antérieure 1, dite première prothèse, et une prothèse postérieure 2, dite deuxième prothèse ; la prothèse antérieure 1 est fixée à une vertèbre telle que L5 ou au sacrum S1, et la prothèse postérieure 2 est fixée à la vertèbre située immédiatement au dessus, telle que L4 ou L5 respectivement.

Par référence aux figures 5 à 18 en particulier, chacune des prothèses 1, 2 comporte un corps 6, 7 et une tige cylindrique d'ancrage 3, 5 intégrée au corps et allongée selon un axe 8, 9.

Par référence aux figures 5 à 8 et 12 à 15, le corps 7 de la prothèse antérieure 1 est en grande partie délimité par deux surfaces opposées : une surface 10 d'appui de la prothèse sur le moignon de l'apophyse articulaire antérieure, et une surface 11 de contact glissant avec la prothèse postérieure (repérée 2) ; comme illustré en particulier sur les vues craniales (figures 5 et 12), la surface 11 comporte deux faces 11a et 11b formant un genre de dièdre dont l'angle d'ouverture 12 vers l'arrière est voisin de 140° pour la prothèse L5-S1 figure 5, et voisin de 120° pour la prothèse L4-L5 figure 12 ; par ailleurs, ces faces 11a, 11b s'étendent le long d'une surface 15a, 15b intermédiaire qui est de révolution, d'axe 13 transverse et dont le rayon de courbure 14 (figures 3 et 4) est voisin de 23 mm ; la portion médiale 11a de la surface 11 de glissement est convexe, tandis que la portion latérale 11b de la surface 11 est plane.

De façon complémentaire, chaque prothèse postérieure 2 figures 9 à 11 et 16 à 18 présente une surface 16 d'appui osseux et une surface 17 d'appui glissant sur la surface 11 de la prothèse 1 correspondante ; la surface 17 comporte une face 17a et une face 17b qui forment un dièdre convexe (figures 9 et 16) dont l'angle d'ouverture 18 est voisin de 220° (figure 9) ou de 240° (figure 16) ; la portion médiale 17a de la surface 17 de glissement est concave, tandis que la portion latérale 17b est sensiblement plane.

La concavité du dièdre 11a, 11b de la prothèse antérieure est dirigée vers l'arrière, tandis que la convexité du dièdre 17a, 17b de la prothèse postérieure est dirigée vers l'avant.

La prothèse postérieure L5-S1 (figure 10) comporte un orifice 19 spécialement prévu pour recevoir une vis d'ancrage supplémentaire (repère 4 figures 1 et 2) de la prothèse dans la lame vertébrale 20.

La fixation des prothèses 1 et 2 est renforcée de par la présence de nervures stabilisatrices 21 (figures 5, 7, 8 notamment), 22, 23, 24 (figures 10 et 17) qui font saillie à la périphérie de la surface d'appui osseux 10, 16 ; dans le cas de la prothèse postérieure 2, les nervures 22 à 24 et la surface 16 délimitent une rainure ou gouttière.

La préparation de l'implantation des prothèses nécessite un abord postérieur de la colonne vertébrale avec exposition de la ou des articulations postérieures à remplacer. Une excision (ou ablation) partielle au moins des apophyses articulaires est nécessaire. Sur la facette (ou apophyse articulaire) antérieure (ou apophyse articulaire supérieure de la vertèbre sous-jacente) seule la surface articulaire est abrasée avec amenuisement du volume global de la facette et réalisation d'un trou d'ancrage sagittal à sa partie inférieure. Sur la facette (ou apophyse articulaire) postérieure (ou apophyse articulaire inférieure de la vertèbre sus-jacente) on pratique une ablation sub-totale avec section oblique vers la lame vertébrale et perpendiculaire sur l'isthme vertébral, et deux trous d'ancrage, l'un sagittal dans l'axe de l'isthme vers le pédicule vertébral, et l'autre transversal dans l'épaisseur de la lame vertébrale, creusé à partir de l'apophyse épineuse.

La prothèse antérieure 1 s'appuie sur le moignon préparé de la facette antérieure avec pénétration de son ergot cylindrique d'ancrage dans le trou d'ancrage. La prothèse présente sur sa face orientée vers l'arrière une surface 11, 11a, 11b articulaire lisse dont la forme se présente comme une section de dièdre ouvert vers l'arrière (ou d'une gorge de poulie), avec un secteur médial étroit et frontal et un secteur latéral étalé et oblique. En coupe horizontale, le secteur médial est frontal et le secteur latéral d'une obliquité qui varie de 50 à 20° du sacrum au sommet de la colonne lombaire. Sur une coupe sagittale, les deux secteurs médial 11a et latéral 11b de cette prothèse ont une convexité tournée vers l'arrière qui correspond à l'arc d'un cercle dont le centre correspond au centre de rotation commun aux deux articulations postérieures et au disque intervertébral du même segment mobile vertébral (le rayon de ce cercle est de 23 mm en moyenne).

La prothèse postérieure 2 présente un premier ergot 3 intégré prévu pour s'engager dans le trou d'ancrage isthmique, ainsi qu'un deuxième ergot 4 qui est séparé du corps de la prothèse et est vissé pour l'ancrage lamaire. La surface articulaire 17, 17a, 17b de glissement dirigée vers l'avant s'appuie intimement sur la surface articulaire 11, 11a, 11b de la prothèse antérieure 1.

Les prothèses 1, 2 peuvent être métalliques ou plastiques ou de tout autre matériau qui s'avèrerait biologiquement compatible. La fixation des prothèses peut être renforcée de ciment plastique.

## Revendications

1. Prothèse (1 ou 2) d'articulation vertébrale postérieure gauche ou droite présentant une surface (11, 11a, 11b ou 17, 17a, 17b) lisse d'appui glissant, **caractérisée en ce que** ladite surface présente une courbure antéro-postérieure sagittale, ainsi que des moyens de guidage en rotation comprenant deux faces ou portions de faces faisant partie de ladite surface lisse d'appui glissant (11a, 11b ou 17a, 17b) et formant sensiblement un dièdre, lesdits moyens de guidage étant en outre aptes à coopérer avec une surface d'appui complémentaire d'une prothèse complémentaire (2 ou 1) munissant une vertèbre adjacente.

2. Prothèse (1 ou 2) selon la revendication 1, dans laquelle lesdites faces ou portions de faces s'étendent le long d'une surface de révolution présentant ladite courbure antéro-postérieure.

3. Prothèse (1 ou 2) selon l'une quelconque des revendications 1 et 2, dans laquelle le rayon de courbure de ladite surface lisse d'appui glissant est situé dans une plage allant de 18 à 28 mm.

4. Prothèse (1 ou 2) selon l'une quelconque des revendications 1 à 3, comportant en outre une surface (10, 16) d'appui osseux et une structure (3, 4, 5, 21 à 24) d'ancrage, et dans laquelle la structure d'ancrage fait saillie sur la surface d'appui osseux.

5. Prothèse (1 ou 2) selon la revendication 4, dans laquelle la structure d'ancrage comporte un organe d'ancrage allongé selon un axe (8, 9) incliné par rapport à la normale à la surface d'appui osseux.

6. Prothèse (1 ou 2) selon la revendication 5, dans laquelle l'organe d'ancrage est de forme sensiblement cylindrique.

7. Prothèse (1 ou 2) selon l'un des revendications 5 ou 6, dans laquelle ledit organe d'ancrage est muni sur sa face externe de rainures ou protubérances.

8. Prothèse (1 ou 2) selon l'une quelconque des revendications 4 à 7, dans laquelle au moins une partie de la structure d'ancrage forme une seule pièce avec le corps de la prothèse.

9. Prothèse (1) selon l'une quelconque des revendications 1 à 8, pour le remplacement de l'apophyse articulaire antérieure, dans laquelle une partie au moins de ladite surface (11, 11a, 11b) lisse d'appui est convexe, ouverte vers l'arrière et courbée selon un rayon (14) allant de 18 à 28 millimètres.

10. Prothèse (1) selon la revendication 9 pour le remplacement de l'apophyse articulaire antérieure, dans laquelle les faces (11a, 11b) du dièdre forment un angle (12) allant de 90° à 160°.

11. Prothèse (2) selon l'une quelconque des revendications 1 à 8, pour le remplacement de l'apophyse articulaire postérieure, dans laquelle une partie au moins de ladite surface (17, 17a, 17b) lisse d'appui est concave, fermée vers l'avant et courbée selon un rayon (14) allant de 18 à 28 millimètres.

12. Prothèse (2) selon la revendication 11 pour le remplacement de l'apophyse articulaire postérieure, dans laquelle les faces (17a, 17b) du dièdre forment un angle (18) allant de 200° à 270°.

13. Dispositif de remplacement d'une articulation vertébrale postérieure gauche ou droite, qui comporte :
- une prothèse (1) de remplacement partiel de l'apophyse articulaire antérieure, dite première prothèse, qui est de préférence conforme à l'une quelconque des revendications 1 à 10, qui comporte une première surface (11, 11a, 11b) lisse d'appui glissant en forme de gorge de poulie, et
- une prothèse (2) de remplacement de l'apophyse articulaire postérieure, dite deuxième prothèse, qui est de préférence conforme à l'une quelconque des revendications 1 à 8, 11 ou 12, et qui comporte une deuxième surface (17, 17a, 17b) lisse d'appui glissant dont une portion au moins est de forme similaire ou complémentaire à celle d'une portion au moins de ladite première surface lisse d'appui glissant de ladite première prothèse, de façon à faciliter un mouvement relatif de rotation sagittale de deux vertèbres adjacentes munies de ces prothèses.

## Claims

1. A left or right posterior vertebral joint prosthesis (1 or 2) presenting a smooth sliding bearing surface (11, 11a, 11b or 17, 17a, 17b), **characterized in that** said surface presents antero-posterior sagittal curvature, and pivoting guide means comprising two faces or face portions forming part of said smooth sliding bearing surface (11a, 11b or 17a, 17b) and substantially forming a V-shape, said pivoting guiding means being further suitable for co-operating with a complementary bearing surface of a complementary prosthesis (2 or 1) fitting an adjacent vertebra.

2. A prosthesis (1 or 2) according to claim 1, in which said faces or face portions extend along a circularly cylindrical surface presenting said antero-posterior curvature.

3. A prosthesis (1 or 2) according to any one of claims 1 and 2, in which the radius of curvature of said smooth sliding bearing surface lies in the range 18 mm to 28 mm.

4. A prosthesis (1 or 2) according to any one of claims 1 to 3, further comprising a bone bearing surface (10, 16) and an anchor structure (3, 4, 5, 21 to 24), and in which the anchor structure projects from the bone bearing surface.

5. A prosthesis (1 or 2), according to claim 4, in which the anchor structure includes an anchor member that is elongate an axis (8, 9) that is inclined relative to the normal of the bone bearing surface.

6. A prosthesis (1 or 2) according to claim 5, in which the anchor member is substantially cylindrical in shape.

7. A prosthesis (1 or 2) according to claim 5 or claim 6, in which said anchor member is provided on its outside surface with grooves or projections.

8. A prosthesis (1 or 2) according to any one of claims 4 to 7, in which at least a portion of the anchor structure is integral with the body of the prosthesis.

9. A prosthesis (1) according to any one of claims 1 to 8, for replacing the anterior joint process, in which at least a portion of said smooth bearing surface (11, 11a, 11b) is convex, rearwardly open, and curved with a radius lying in the range 18 mm to 28 mm.

10. A prosthesis (1) according to claim 9, for replacing the anterior joint process, in which the faces (11a, 11b) of the V-shape form a dihedral angle (12) lying in the range 90° to 160°.

11. A prosthesis (2) according to any one of claims 1 to 10, for replacing the posterior joint process, in which at least a portion of said smooth bearing surface (17, 17a, 17b) is concave, forwardly closed, and curved with a radius (14) lying in the range 18 mm to 28 mm.

12. A prosthesis (2) according to claim 11, for replacing the posterior joint process, in which the faces (17a, 17b) of the V-shape form a dihedral angle (18) lying in the range 200° to 270°.

13. A device for replacing a left or right posterior vertebral joint, the device comprising:
· a prosthesis (1) for partially replacing the anterior joint process, referred to as the "first" prosthesis, which prosthesis is preferably in accordance with any one of claims 1 to 10, and has a first smooth sliding bearing surface (11, 11a, 11b) in the form of a pulley groove; and
· a prosthesis (2) for replacing the posterior joint process, referred to as the "second" prosthesis, which is preferably in accordance with any one of claims 1 to 8, 11, or 12, and which includes a second smooth sliding bearing surface (17, 17a, 17b), of which at least a portion is similar or complementary in shape to at least a portion of said first smooth sliding bearing surface of said first prosthesis, so as to facilitate relative sagittal pivoting movement between two adjacent vertebrae fitted with these prostheses.

## Patentansprüche

1. Prothese (1 oder 2) für ein linkes oder rechtes hinteres Wirbelgelenk, die eine glatte Fläche (11, 11a, 11b oder 17, 17a, 17b) zur gleitenden Anlage aufweist, **dadurch gekennzeichnet, daß** die genannte Fläche eine antero-posteriore Sagittalkrümmung sowie Drehführungsmittel aufweist, die zwei Flächen oder Flächenabschnitte umfassen, welche zu der glatten Gleitanlagefläche (11a, 11b oder 17a, 17b) gehören und im wesentlichen ein Dieder bilden, wobei die Führungsmittel ferner geeignet sind, mit einer ergänzenden Anlagefläche einer ergänzenden Prothese (2 oder 1), mit der ein benachbarter Wirbel versehen ist, zusammenzuwirken.

2. Prothese (1 oder 2) nach Anspruch 1, wobei die Flächen oder Flächenabschnitte sich entlang einer die genannte antero-posteriore Krümmung aufweisenden Rotationsfläche erstrecken.

3. Prothese (1 oder 2) nach einem der Ansprüche 1 und 2, wobei der Krümmungsradius der glatten Gleitanlagefläche in einem Bereich zwischen 18 und 28 mm liegt.

4. Prothese (1 oder 2) nach einem der Ansprüche 1 bis 3, die ferner eine Knochenanlagefläche (10, 16) und eine Verankerungsstruktur (3, 4, 5, 21 bis 24) aufweist und wobei die Verankerungsstruktur an der Knochenanlagefläche vorspringt.

5. Prothese (1 oder 2) nach Anspruch 4, wobei die Verankerungsstruktur ein längliches Verankerungsorgan entlang einer Achse (8, 9), die gegenüber der Normalen zur Knochenanlagefläche geneigt ist, umfaßt.

6. Prothese (1 oder 2) nach Anspruch 5, wobei das Verankerungsorgan im wesentlichen zylinderförmig ausgebildet ist.

7. Prothese (1 oder 2) nach einem der Ansprüche 5 oder 6, wobei das Verankerungsorgan an seiner Außenseite mit Nuten oder Vorsprüngen versehen ist.

8. Prothese (1 oder 2) nach einem der Ansprüche 4 bis 7, wobei wenigstens ein Teil der Verankerungsstruktur mit dem Körper der Prothese ein einziges Teil bildet.

9. Prothese (1) nach einem der Ansprüche 1 bis 8, für den Ersatz des vorderen Gelenkfortsatzes, wobei wenigstens ein Teil der glatten Anlagefläche (11, 11a, 11b) konvex, nach hinten offen und mit einem Radius (14) zwischen 18 und 28 mm gekrümmt ist.

10. Prothese (1) nach Anspruch 9, für den Ersatz des vorderen Gelenkfortsatzes, wobei die Flächen (11a, 11b) des Dieders einen Winkel (12) zwischen 90° und 160° bilden.

11. Prothese (2) nach einem der Ansprüche 1 bis 8, für den Ersatz des hinteren Gelenkfortsatzes, wobei wenigstens ein Teil der glatten Anlagefläche (17, 17a, 17b) konkav, nach vorne geschlossen und mit einem Radius (14) zwischen 18 und 28 Millimetern gekrümmt ist.

12. Prothese (2) nach Anspruch 11, für den Ersatz des hinteren Gelenkfortsatzes, wobei die Flächen (17a, 17b) des Dieders einen Winkel (18) zwischen 200° und 270° bilden.

13. Vorrichtung für den Ersatz eines linken oder rechten hinteren Wirbelgelenks, die folgendes umfaßt:
- eine Prothese (1) für den teilweisen Ersatz des vorderen Gelenkfortsatzes, sogenannte erste Prothese, vorzugsweise entsprechend einem der Ansprüche 1 bis 10, die eine erste glatte Fläche (11, 11a, 11b) zur gleitenden Anlage in Form einer Scheibenrille aufweist, sowie
- eine Prothese (2) für den Ersatz des hinteren Gelenkfortsatzes, sogenannte zweite Prothese, vorzugsweise entsprechend einem der Ansprüche 1 bis 8, 11 oder 12, die eine zweite glatte Fläche (17, 17a, 17b) zur gleitenden Anlage aufweist, von welcher wenigstens ein Abschnitt eine ähnliche oder ergänzende Form wie wenigstens ein Abschnitt der ersten glatten Fläche zur gleitenden Anlage der ersten Prothese aufweist, so daß eine relative sagittale Drehbewegung von zwei mit diesen Prothesen versehenen benachbarten Wirbeln erleichtert wird.
